# EUROPÄISCHE PATENTSCHRIFT

(11) **EP 0 502 815 B1**
(45) Veröffentlichungstag und Bekanntmachung des Hinweises auf die Patenterteilung: **24.04.1996**
(21) Anmeldenummer: 92810079.1
(22) Anmeldetag: 05.02.1992
(51) Int. Cl.: A61F 2/38, A61F 2/30

(54) **Schraubenmutter für Implantatverschraubung**
Nut for threaded connection in an implant
Ecrou pour raccord vissé d'un implant

(30) Priorität: 07.03.1991 CH 692/91
(43) Veröffentlichungstag der Anmeldung: 09.09.1992
(73) Patentinhaber: SULZER Medizinaltechnik AG, CH-8404 Winterthur (CH); PROTEK AG, CH-3110 Münsingen-Bern (CH)
(72) Erfinder: Moser, Walter, CH-3037 Herrenschwanden (CH); Willi, Roland, CH-8413 Neftenbach (CH)
(74) Vertreter: Hammer, Bruno, Dr.

(56) Entgegenhaltungen:
- DE-A- 3 017 953
- US-A- 3 053 357
- US-A- 4 822 366
- US-A- 4 936 853

## Beschreibung

Die Erfindung handelt von einer Implantatverschraubung mit Schraubenmutter gemäss Oberbegriff von Anspruch 1.

Im Gegensatz zu starren Verbindungen des Maschinenbaus wäre es bei bestimmten Verbindungen von Implantatskomponenten durchaus vorteilhaft, wenn Mikrobewegungen zwischen den Komponenten möglich sind, um der Elastizität des eingewachsenen Knochengewebes und den mittelfristigen Veränderungen im Knochengewebe Rechnung zu tragen. So zeigt die EP-OS 0 333 642 A1 eine Verankerungsplatte für die tibialen Lagerflächen, die mit Verankerungszapfen durch eine von aussen lösbare Schraubverbindung verbunden ist.

Ein Nachteil der Verbindung liegt darin, dass sie - schon wegen der Sicherung der Schraubverbindung - starr ausgeführt ist und Bewegungen der Tibiaplatte zum Knochengewebe uneingeschränkt an die Verankerungszapfen weitergibt.

Hier schafft die Erfindung Abhilfe. Sie löst die Aufgabe einen im Knochengewebe verankerten Verankerungszapfen so durch eine Schraubverbindung in der Tibiaplatte zu sichern, dass Mikroschwenkbewegungen zwischen Verankerungszapfen und der Tibiaplatte stattfinden können.

Gemäss der Erfindung wird die Aufgabe mit dem kennzeichenden Teil des Anspruchs 1 gelöst.

Der Vorteil der Erfindung besteht darin, dass für einen begrenzten Bereich mit dem Auftreten eines Biegemomentes in der Einspannung des Verankerungszapfens eine mit dem Biegemoment wachsende Schwenkbewegung auftritt, ohne dass sich langfristig die Elemente der Verschraubung von der Tibiaplatte lösen können. Die abhängigen Ansprüche 2 und 3 beziehen sich auf vorteilhafte Weiterbildungen der Erfindung.

Im folgenden wird die Erfindung anhand von einem Ausführungsbeispiel beschrieben. Es zeigt:
- Fig. 1: schematisch einen Schnitt durch die Verankerung eines Verankerungszapfens in einer Tibiaplatte mit einer Schraubverbindung; und
- Fig. 2: schematisch die perspektivische Ansicht einer in Fig. 1 gezeigten Schraubenmutter.

Die Figuren zeigen eine Schraubenmutter für Implantatsverschraubungen mit einer radial vorstehenden Anpressschulter, die axial gegen ein Gehäuseteil presst, mit einem Innengewinde passend zu einem Schraubenteil und mit Formelementen auf der dem Gehäuseteil abgekehrten Seite, um ein Werkzeug für das Anziehen der Schraubenmutter einzusetzen. Erfindungegemäss sind an der Schraubenmutter in axialer Richtung vorstehende Blattfedern angeformt, die als Verklinkungsnocken enden. Beim Einpressen der Schraubenmutter am Gehäuseteil rasten die Verklinkungsnocken mit dem Aufliegen der Anpressschulter in einer Aussparung am Gehäuseteil ein, um ein Ablösen der Schraubenmutter vom Gehäuseteil zu verhindern.

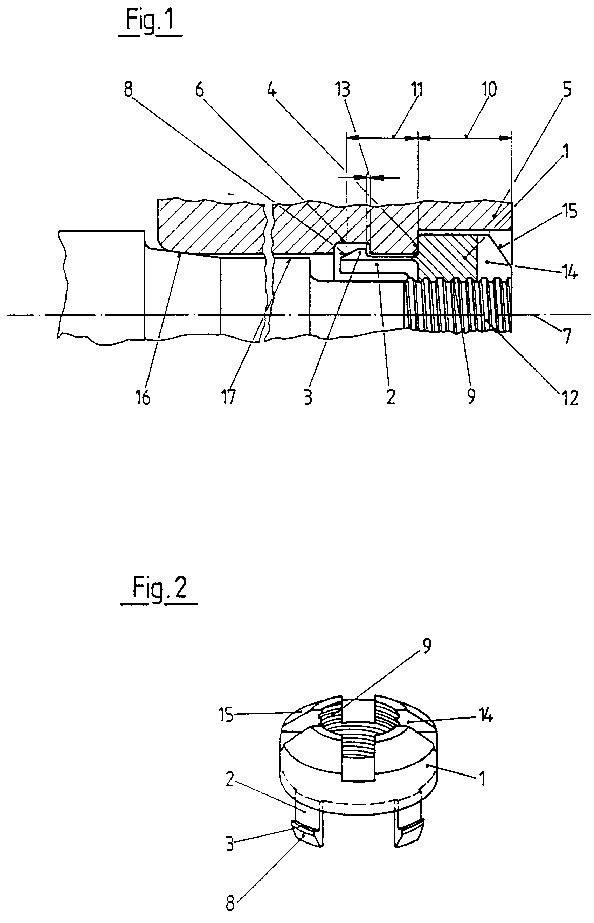

In Figur 1 ist ein Verankerungszapfen in einer zylindrischen Bohrung einer als Tibiaplatte aurgebildeten Gehäuseteils 5 eingesteckt und axial durch eine Schraubenmutter 1 gesichert. Der Zapfen sitzt mit einem kurzen Konusteil 16 am Eintritt der Bohrung auf, während der daran anschliessende Zylinderteil 17 Spiel zur Bohrung aufweist, um bezogen auf die Einspannung am Konusteil 16 kleine Schwenkbewegungen durchführen zu können. Der Verankerungszapfen endet an der ässeren Seite der Tibiaplatte als Gewindebolzen mit einem Schraubenteil 12, auf dem die Schraubenmutter 1 mit Innengewinde 9 reitet.

In Figur 2 ist eine Schraubenmutter 1 zu sehen, bei der vier in axialer Richtung vorstehende Blattfedern 2 bestehen, die als Verklinkungsnocken 3 enden, wobei diese Verklinkungsnocken in Einsteckrichtung schräge oder verrundete Auflaufflächen 8 und in Aurziehrichtung steile Verklinkungsflächen besitzen.

Beim Einfahren der Schraubenmutter 1 in den Gehäuseteil 5 werden die Blattfedern mit dem Auflaufen der Auflaufflächen 8 radial nach innen vorgespannt, bis die Verklinkungsnocken spätestens mit dem Aufliegen der Anpresschulter 4 in einer Aussparung 6 am Gehäuseteil 5 einrasten. Mit am Gehäuseteil 5 anliegender Anpressschulter 4 ergibt sich in Richtung der Schraubenachse 7 ein Spiel 13 zwischen Verklinkungsnocken 3 und Gehäuseteil 5, dar so gross bemessen ist, dass die verklinkte Schraubenmutter 1 im Gehäuseteil 5 drehbar gelagert ist. Die Schraubenmutter 1 beritzt auf der dem Gehäuseteil abgekehrten Seite Formelemente 14 in Form von Schlitzen in die ein Werkzeug eingreifen kann, um die Mutter anzuziehen. Auch wenn die Schraubenmutter 1 bei der Erstverschraubung mit dem Verankerungszapfen satt angezogen wurde, wird nicht angenommen, dass die axiale Vorspannung erhalten bleibt. Mikrobewegungen und ein Setzen im Konusteil lockern die Verbindung zwischen Schraubenteil 12 und Innengewinde 9, so dass die geplanten Schwenkbewegungen des Verankerungszapfens gegenüber dem Gehäuseteil 5 stattfinden können, ohne dass sich die Schraubenmutter 1 wegen der Verklinkung unzulässig vom Gehäuseteil 5 löst.

Die Schraubenmutter 1 kann im Gehäuseteil 5 vormontiert werden, indem sie axial im Gehäuseteil eingepresst wird, bis die Anpressschulter 4 aufritzt. Nach dem Einschlagen des Verankerungszapfens im Tibiateil wird das als Tibiaplatte ausgebildete Gehäuseteil 5 über den Schraubenteil 12 eingefahren bis das Innengewinde 9 aufsitzt und weiter unter Drehen der Schraubenmutter 1 bis in seine Endlage gebracht.

Eine andere Möglichkeit besteht darin, den Verankerungszapfen fast fertig einzuschlagen, die Tibiaplatte über den Schraubenteil 12 aufzusetzen bis der Konusteil 16 greift und beide Teile gemeinsam bis zur Endlage einzurchlagen. Anschliessend wird die Schraubenmutter 1 aufgesetzt und entlang dem Schraubenteil 12 eingedreht bis die Anpressschulter 4 am Gehäuseteil anschlägt und eine Verklinkung mit den Verklinkungsnocken 3 stattfindet. In Figur 1 schliesst der Schraubenteil 12 mit der Aussenfläche der Tibiaplatte ab. Die Gegenfläche zur Anpressschulter 4 ist um eine Länge 10 nach innen zurückgesetzt. Der axiale Berührungrabstand 11 zwischen dem Innengewinde 9 und dem Punkt der Auflaufflächen 8, der als erster am Gehäuseteil 5 aufläuft, ist deutlich kleiner als die Länge 10, damit die Einpressbewegung durch das greifende Innengewinde 9 und durch ein Drehmoment an der Schraubenmutter 1 erfolgt. Das Eindrehen der Schraubenmutter erfolgt durch ein Hilfswerkzeug, das sich an einer Anschrägung 15 der Schraubenmutter zentrieren kann und dar in die Schlitze 14 eingreift, um dar notwendige Drehmoment zu übertragen.

## Patentansprüche

1. Implantatverschraubung mit einer Schraubenmutter (1), die eine radial vorstehende Anpressschulter (4) aufweist, die beim Verschrauben axial gegen ein Gehäuseteil (5) beispielsweise einer Tibiaplatte presst, mit einem Innengewinde (9) passend zu einem Schraubenteil des Gehäuseteils (5) und mit Formelementen (14) auf der dem Gehäuseteil (5) abgekehrten Seite, um ein Werkzeug für das Anziehen der Schraubenmutter (1) einzusetzen, dadurch gekennzeichnet, dass an der Schraubenmutter (1) in axialer Richtung vorstehende Blattfedern (2) angeformt sind, die als Verklinkungsnocken (3) enden, welche mit dem Aufliegen der Anpressschulter (4) am Gehäuseteil (5) in einer Aussparung (6) am Gehäuseteil (5) derart einrasten, dass einerseits ein Ablösen der Schraubenmutter (1) vom Gehäuseteil (5) verhindert wird und andererseits die Verklinkungsnocken (3) in axialer Richtung zu ihrer Gegenfläche am Gehäuseteil (5) ein Spiel (13) aufweisen, um die mit dem Gehäuseteil verklinkte Schraubenmutter (1) gegenüber dem Gehäuseteil (5) drehbar zu machen.

2. Implantatverschraubung nach Anspruch 1, dadurch gekennzeichnet, dass die Verklinkungsnocken (3) mit schräger oder verrundeter Auflauffläche (8) versehen sind, über die die Blattfedern (2) beim Einfahren in das Gehäuseteil (5) vorgespannt werden, um beim Aufliegen der Anpressschulter (4) eine Verklinkungsbewegung auszuführen.

3. Implantatverschraubung nach einem der Ansprüche 1 und 2, dadurch gekennzeichnet, dass von der Anpressschulter (4), in axialer Richtung gemessen, die Länge (10) der eigentlichen Mutter mit Gewindeteil grösser ist als der Berührungsabstand (11) zum Gehäuseteil (5), bei dem die Deformation der Blattfedern (2) einsetzt, damit die Deformation der Blattfedern (2) durch eine Schraubbewegung des greifenden Innengewindes (9) vorgenommen werden kann, wenn der Schraubenteil (12) in montierter Endlage nicht axial über die Schraubenmutter (1) vorstehen soll.

## Claims

1. The screwing of implants together by a screw-nut (1) exhibiting a radially projecting thrust-shoulder (4) which during the screwing together passes axially against a housing-part (5) of, for example, a tibia-plate, and having an internal thread (9) which fits a screw part of the housing-part (5), together with elements (14) moulded onto the side facing away from the housing-part (5) for fitting a tool for tightening the screw-nut (1), characterised in that leaf springs (2) projecting in the axial direction are moulded onto the screw-nut (1) and end in latching-in dogs (3) which upon the thrust-shoulder (4) making contact with the housing-part (5) snap into a recess (6) in the housing-part (5) in such a way that on the one hand loosening of the screw-nut (1) from the housing-part (5) is prevented, and on the other hand the latching-in dogs (3) exhibit a clearance (13) in the axial direction from their counterface on the housing-part (5) in order to make it possible to turn the screw-nut (1) with respect to the housing-part (5) while latched into the housing-part.

2. The screwing of implants together as in Claim 1, characterised in that the latching-in dogs 93) are provided with oblique or rounded run-up faces (8) by which upon the leaf springs (2) being run into the housing part (5) they are prestressed in order to execute a latching-in motion, upon the thrust-shoulder (4) making contact.

3. The screwing of implants together as in one of the Claims 1 and 2, characterised in that measured from the thrust-shoulder (4) in the axial direction the length (10) of the actual nut with the thread part is greater than the distance (11) from the point of contact with the housing part (5) at which deformation of the leaf springs (2) starts, so that deformation of the leaf springs (2) may be performed by a screwing motion of the engaging internal thread (9) if the screw part (12) must not project axially beyond the screw-nut 91) in the final mounted position.

## Revendications

1. Raccord vissé d'implant avec un écrou (1), qui présente un épaulement de pression (4) faisant saillie radialement, qui lors du vissage appuie axialement contre une partie de boîtier (5) par exemple d'une plaque de tibia, avec un filetage intérieur (9) adapté à une partie de vis de la partie de boîtier (5) et avec des éléments de forme (14) sur le côté opposé à la partie de boîtier (5), afin d'insérer un outil pour le serrage de l'écrou (1), caractérisé en ce que sur l'écrou (1) sont formés des ressorts à lames (2) dépassant axialement, qui se terminent en cames d'encliquetage (3), qui lorsque l'épaulement de pression (4) repose sur la partie de boîtier (5), s'encliquettent dans une découpe (6) de la partie de boîtier (5) de manière que d'une part l'écrou (1) ne puisse se détacher de la partie de boîtier (5) et d'autre part que les cames d'encliquetage (3) présentent axialement vers leur contre-surface sur la partie de boîtier (5), un jeu (13) afin de rendre tournant par rapport à la partie de boîtier (5), l'écrou (1) encliqueté avec la partie de boîtier.

2. Raccord vissé d'implant selon la revendication 1, caractérisé en ce que les cames d'encliquetage (3) sont pourvues d'une surface de montée (8) oblique ou arrondie, par laquelle les ressorts à lames (2) sont précontraints lors de leur introduction dans la partie de boîtier (5), afin d'exécuter un mouvement d'encliquetage lorsque l'épaulement de pression (4) repose sur celle-ci.

3. Raccord vissé d'implant selon l'une des revendications 1 et 2, caractérisé en ce que de l'épaulement de pression (4), mesurée dans la direction axiale, la longueur (10) de l'écrou proprement dit avec partie filetée, est supérieure à la distance de contact (11) avec la partie de boîtier (5), à laquelle s'instaure la déformation des ressorts à lames (2), afin que la déformation des ressorts à lames (2) puisse être assurée par un mouvement de vissage du filetage intérieur (9) agissant, lorsque la partie de vis (12) en position terminale montée ne doit pas dépasser axialement de l'écrou (1).
